# EUROPÄISCHE PATENTSCHRIFT

(11) **EP 0 590 557 B1**
(45) Veröffentlichungstag und Bekanntmachung des Hinweises auf die Patenterteilung: **14.08.1996**
(21) Anmeldenummer: 93115523.8
(22) Anmeldetag: 25.09.1993
(51) Int. Cl.: C07C 69/157, C07C 67/297

(54) **Verfahren zur Herstellung von 4-Acetoxystyrol**
Process for preparing acetoxystyrene
Procédé pour la préparation de l'acétoxystyrène

(30) Priorität: 01.10.1992 DE 4233039
(43) Veröffentlichungstag der Anmeldung: 06.04.1994
(73) Patentinhaber: BASF Aktiengesellschaft, 67063 Ludwigshafen (DE)
(72) Erfinder: Brudermueller, Martin, Dr., D-6800 Mannheim 1 (DE); Merger, Franz, Dr., D-6710 Frankenthal (DE)

(56) Entgegenhaltungen:
- EP-A- 0 465 147
- US-A- 2 276 138
- US-A- 5 151 546
- CHEMICAL ABSTRACTS, Band 52, Nr. 20, 25. Oktober 1958, Columbus, Ohio, USA; B. CORSON et al: "Preparation of vinylphenols and isoprenylphenols", Zusammenfassung Nr. 17 152e-h

## Beschreibung

Die vorliegende Erfindung betrifft ein Verfahren zur Herstellung von 4-Acetoxystyrol aus 1-(4-Acetoxyphenyl)ethylcarboxylaten an acidwirkenden Katalysators in einem inerten Wärmeträger in Gegenwart eines Polymerisationsinhibitors bei erhöhten Temperaturen und vermindertem Druck.

Die Herstellung von 4-Hydroxystyrol durch katalytische Dehydrierung von 4-Hydroxyethylbenzol ist aus der EP-A-128 984 bekannt.

In J.Org.Chem. 23, 544 bis 549 (1958) ist die Herstellung von 4-Acetoxystyrol aus Phenol in vier Verfahrensstufen beschrieben: Acetylierung von Phenol zu 4-Hydroxyacetophenon, Veresterung zu 4-Acetoxyacetophenon, Hydrierung zu 1-(4-Acetoxyphenyl)ethanol und Dehydratisierung zu 4-Acetoxystyrol. Die Dehydratisierung von 1-(4-Acetoxyphenyl)-ethanol erfolgt dabei in der Gasphase an aktiviertem Aluminiumoxid bei 350°C oder auch in der Flüssigphase in Gegenwart von festem Kaliumhydrogensulfat.

Beide Verfahren führen jedoch besonders bei Durchführung im technischen Maßstab infolge hoher Polymerisationsneigung des Produktes zu niederen Ausbeuten und sind daher unwirtschaftlich.

EP-A-355 983 beschreibt die Herstellung von 4-Acetoxystyrol durch Dehydratisierung von 1-(4-Acetoxyphenyl)ethanol in einem Dünnschichtverdampfer unter Einhaltung sehr kurzer Verweilzeiten. In Gegenwart von Polymerisationsinhibitoren und katalytischen Mengen Kaliumhydrogensulfat werden bei unvollständigen Umsätzen unbefriedigende Ausbeuten erzielt.

Die im kontinuierlichen Betrieb der Reaktoren auftretende Belegung der Reaktoroberfläche mit Polymeren kann nicht wirksam verhindert werden und steht damit einer wirtschaftlichen Nutzung des Verfahrens entgegen.

Ein weiterer Nachteil aller bisher beschriebenen Verfahren zur Dehydratisierung von 1-(4-Acetoxyphenyl)ethanol ist der Anfall zweiphasiger Reaktionsausträge durch die Bildung stöchiometrischer Mengen Wasser. Die Inhomogenität der Reaktionsgemische verursacht Probleme in der Reaktionsführung.

In US-A-5 041 614 ist die Dehydratisierung von 1-(4-Acetoxyphenyl)-ethanol in Gegenwart von Carbonsäureanhydriden, eines Dehydratisierungskatalysators und von Polymerisationsinhibitoren beschrieben.

Das entstehende Wasser, das unter anderem die Bildung von Nebenprodukten wie Polymere begünstigt, wird durch den Zusatz eines Carbonsäureanhydrids unter Bildung von Carbonsäuren zurückgedrängt.

Durch diese Maßnahme kann die Bildung von polymeren Nebenprodukten jedoch nicht unterbunden werden. Vielmehr reichern sich hochsiedende Rückstände im Reaktionsraum an. Die Anreicherung von polymeren Nebenprodukten führt zu einer Verminderung der Ausbeute an Wertprodukt und zu einem Abfall der Selektivität für 4-Acetoxystyrol. Die Polymerprodukte müssen daher im Verlaufe der Umsetzung mit erheblichen Aufwand kontinuierlich aus dem Reaktor entfernt werden.

Das Verfahren nach US-A-5 041 614 verlangt also gegenüber dem vorgängigen Stand der Technik einen höheren apparativen Aufwand und bringt bezüglich der erzielten Ausbeute an 4-Acetoxystyrol keine wesentlichen Vorteile.

Die thermische Spaltung von 1-(4-Acetoxyphenyl)ethylacetat (APEA) zu 4-Acetoxystyrol in der Gasphase bei Temperaturen um 480°C ist aus der US-A-2 276 138 bekannt. Die Herstellung von APEA erfolgt durch Veresterung von 1-(4-Acetoxyphenyl)ethanol mit Acetylchlorid.

Die Ausbeute verbleibt infolge der Bildung von polymeren teerartigen Nebenprodukten auf dem niedrigen Niveau von ca. 50 %. Das Verfahren konnte daher ebenfalls keinen Eingang in die Technik finden.

Der vorliegenden Erfindung lag daher die Aufgabe zugrunde, den zuvor genannten Nachteilen abzuhelfen.

Demgemäß wurde ein neues und verbessertes Verfahren zur Herstellung von 4-Acetoxystyrol der Formel I 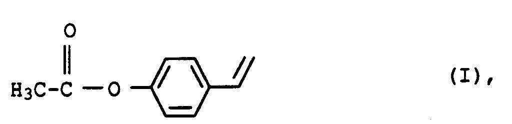 gefunden, welches dadurch gekennzeichnet ist, daß man 1-(4-Acetoxyphenyl)ethylcarboxylate der allgemeinen Formel II 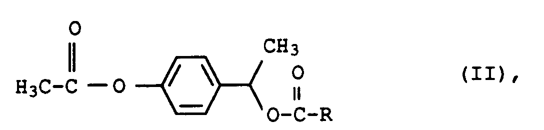 in der R C₁- bis C₁₀-Alkyl, C₃- bis C₆-Cycloalkyl, Aryl oder C₇- bis C₁₀-Aralkyl bedeutet, in Gegenwart eines sauren Katalysators und eines Polymerisationsinhibitors in einem inerten Wärmeträger bei Temperaturen von 160 bis 250°C und einem Druck von 0,1 bis 300 mbar umsetzt.

Das erfindungsgemäße Verfahren läßt sich wie folgt durchführen:

Die 1-(4-Acetoxyphenyl)ethylcarboxylate II können in reiner Form, gegebenenfalls als Schmelze, oder in der Form einer Lösung in inerten Lösungsmitteln, vorzugsweise in einer bei der Veresterung anfallenden Lösung in der korrespondierenden Carbonsäure in der Flüssigphase vorzugsweise in inerten Wärmeträgern bei Temperaturen von 160 bis 250°C, bevorzugt 180 bis 210°C und einem Druck von 0,1 bis 300 mbar, bevorzugt 1 bis 100 mbar, besonders bevorzugt 5 bis 80 mbar in Gegenwart eines sauren Katalysators und eines Polymerisationsinhibitors umgesetzt werden.

Der saure Katalysator kann wie die Polymerisationsinhibitoren im inerten Wärmeträger vorgelegt oder im Reaktionsverlauf kontinuierlich oder diskontinuierlich zum Reaktionsgemisch zugegeben werden.

Die Reaktion wird vorteilhaft in einem Reaktionskessel mit aufgesetzter Destillationskolonne durchgeführt. Vorteilhaft ist es, den inerten Wärmeträger, den Spaltungskatalysator und Polymerisationsinhibitor im Reaktionskessel vorzulegen und das Gemisch auf Reaktionstemperatur vorzuheizen. Nach Einstellen des Betriebsdruckes mittels angeschlossener Vakummanlage am Kolonnenkopf wird das Carboxylat kontinuierlich oder semikontinuierlich zudosiert. Am Kolonnenkopf wird im gleichen Umfang ein einphasiges Gemisch aus 4-Acetoxystyrol und Carbonsäure gasförmig abgenommen und in einer Vorlage kondensiert.

Bei Eindosieren des Produktes in das flüssige Reaktionsmedium, vorzugsweise über ein Tauchrohr, ist es möglich durch Wahl des Reaktionsdruckes, der Temperatur und des Rücklaufverhältnisses bei der kontinuierlichen Destillation von 4-Acetoxystyrol aus dem Reaktionsgemisch bei hoher Raum-Zeit-Ausbeute die Stationärkonzentration von Edukt und Produkt so gering zu halten, daß Nebenreaktionen wie die Bildung von Polymeren weitgehend unterdrückt werden.

Bei diskontinuierlicher Fahrweise kann der inerte Wärmeträger mit den darin homogen gelösten polymeren Nebenprodukten nach Abschluß der Reaktion entsorgt werden oder der inerte Wärmeträger destillativ abgetrennt und wieder eingesetzt werden. In kontinuierlicher Fahrweise wird fortlaufend ein Teil des Wärmeträgers kontinuierlich entfernt und durch neuen Wärmeträger ersetzt, um den Polymerengehalt auf niedrigem Niveau zu halten.

Als 1-(4-Acetoxyphenyl)ethylcarboxylate II eignen sich Ester von 1-(4-Acetoxyphenyl)ethanol mit aliphatischen oder aromatischen Carbonsäuren, die in freier Form unter den Reaktionsbedingungen abdestillierbar und vom Produkt Acetoxystyrol durch fraktionierende Destillation abtrennbar sind, vorzugsweise Essigsäure, Propionsäure, Buttersäure, Isobuttersäure und Benzoesäure, sowie substituierte Carbonsäuren wie Methoxyessigsäure kommen in Betracht. Besonders bevorzugt wird der Ester der Essigsäure.

Die Ester können in bekannter Weise durch Umsetzung des 1-(4-Acetoxyphenyl)ethanols mit Carbonsäuren, bzw. den Anhydriden oder Chloriden der genannten Säuren hergestellt werden (Organikum, VEB Deutscher Verlag der Wissenschaften, 1981, S. 498-505).

Als inerte Wärmeträger eignen sich inerte hochsiedende Wärmeträger wie technische Öle mit einem Siedebereich, der über dem des 4-Acetoxystyrols liegt, beispielsweise Gasöl, Vakuumöl, Heizöl technisches Weißöl, Mineralöle, geschmolzenes Parrafinwachs, Silikonöl oder auch Alkylaromaten, wie z.B. Dodecylbenzol. Auch andere unter den Reaktionsbedingungen inerte Flüssigkeiten oder Gemische mit ausreichend niedriger Viskosität, die gute Rührbarkeit bei Reaktionstemperatur gewährleistet können eingesetzt werden. Besonders bevorzugt werden Heizöle, die durch ihre kontinuierliche Zudosierung und Entfernung aus dem Reaktionsraum den kontinuierlichen Abzug von Polymeren in noch niedriger Konzentration ermöglichen und mit ihnen zusammen zur Energieerzeugung genutzt werden können. Der Abzug der Polymeren in relativ niedriger Konzentration - bezogen auf den Wärmeträger im Reaktor - gewährt gleichbleibend gute Ausbeuten an 4-Acetoxystyrol.

Als Polymerisationsinhibitoren eignen sich kommerziell verfügbare Verbindungen wie beispielsweise tert.-Butyl-brenzkatechin, Hydrochinon, Hydrochinonmonomethylether oder Phenothiazin, vorzugsweise Gemische aus Phenothiazin und Hydrochinonmonomethylether, die in einer Konzentration von 0,01 bis 5 Gew.-% bezogen auf den inerten Wärmeträger zugesetzt werden. Der Inhibitor kann im inerten Wärmeträger vorgelegt oder mit dem Einsatzstoff kontinuierlich zudosiert werden.

Als saure Katalysatoren eignen sich oxidische Feststoffkatalysatoren, wie beispielsweise Aluminiumoxid, Silicagel, Titanoxid, Zeolithe und/oder Heteropolysäuren, in Konzentrationen von 0,1 bis 50 Gew.-%, vorzugsweise 3 bis 10 Gew.-% bezogen auf den inerten Wärmeträger.

Die sauren Katalysatoren können als Tabletten, Splitt oder vorzugsweise als Pulver eingesetzt werden. Technisch vorteilhaft ist der Einsatz als Pulver, so daß der Katalysator als Suspension im inerten Wärmeträger vorliegt.

Als Katalysatoren sind ferner auch organische Säuren beispielsweise p-Toluolsulfonsäure oder Mineralsäuren geeignet.

Oxidische Feststoffkatalysatoren können vom inerten Wärmeträger abgetrennt und gegebenenfalls bei nachlassender Aktivität nach einer thermischen Behandlung bei 350 bis 500°C regeneriert und der Reaktion wieder zurückgeführt werden.

Der Substituent R in Verbindung II hat folgende Bedeutung:
- R: - C₁- bis C₁₀-Alkyl, Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl und iso-Decyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₃- bis C₆-Cycloalkyl wie Cyclopropyl, Cyclobutyl, Cyclopentyl und Cyclohexyl, bevorzugt Cyclopentyl und Cyclohexyl,
- Aryl wie Phenyl, 1-Naphthyl und 2-Naphthyl, bevorzugt Phenyl,
- C₇- bis C₁₀-Aralkyl, bevorzugt C₇- bis C₁₀-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl.

Polymere aus Acetoxy- bzw. Hydroxystyrolen finden Verwendung unter anderem in Klebstoffen, Beschichtungen und im besonderen in Photoresistfilmen (siehe z. B. J. Photopolym. Sci. Technol. 3, 347 (1990).

### Beispiele

Die nachstehenden Beispiele wurden in einer Apparatur durchgeführt, die aus einem beheizten Rührkolben mit aufgesetzter Kolonne und Kondensator sowie Destillatvorlage mit Anschluß an eine Vakuumpumpe zur kontrollierten Einstellung des gewünschten Druckes, Dosierung des Einsatzstoffes über Pumpe via Tauchrohr in die Flüssigphase des Reaktionsraumes bestand.

### Beispiel 1

In einen 500 ml-Rundkolben mit Destillationsaufsatz wurden 300 g Marlothermöl, 45 g pulverförmiges Al₂O₃/SiO₂ (80 Gew.-%/20 Gew.-%) und je 3 g Phenothiazin und Hydrochinonmonomethylether vorgelegt. Bei einer Öltemperatur von 200°C und einem Druck von 14 mbar dosierte man innerhalb von 2 h eine Schmelze von 215 g 1-(4-Acetoxyphenyl)ethylacetat (APEA) (81 Gew.-% APEA, 19 Gew.-% Essigsäure) in das heiße Öl und kondensiert kontinuierlich bei einer Temperatur von ca. 100°C am Kolonnenkopf 177 g einphasiges Destillat mit folgender Zusammensetzung:
- 23,7 Gew.-%: Essigsäure
- 61,1 Gew.-%: 4-Acetoxystyrol
- 1,0 Gew.-%: 4-Hydroxystyrol
- 10,7 Gew.-%: 1-(4-Acetoxyphenyl)ethylacetat

Die Ausbeute an 4-Acetoxystyrol betrug 85 % bei einem Umsatz von 89 % bezogen auf APEA (Selektivität 95 %).

### Beispiele 2 bis 4

Die Durchführung erfolgte analog Beispiel 1.

Die Ergebnisse sind der folgenden Tabelle zu entnehmen.

**Tabelle**

| Katalysator | Reaktionstemp. [°C] | Druck [mbar] | Dosierung [ml/min] | Ausbeute [%] | Umsatz [%] | Selektivität [%] |
|---|---|---|---|---|---|---|
| Al₂O₃/SiO₂ (80/20) 10 Gew.-% | 230 | 27 | 0,7 | 80,0 | 96,0 | 83,3 |
| Al₂O₃/SiO₂ (80/20) 10 Gew.-% | 210 | 18 | 0,7 | 84,3 | 95,1 | 88,7 |
| Al₂O₃/SiO₂ (80/20) 10 Gew.-% | 180 | 10 | 0,7 | 75,1 | 79,2 | 94,8 |

### Beispiel 5

In einen 500 ml-Rundkolben mit Destillationsaufsatz wurden 400 g Marlothermöl, 30 g pulverförmiges Al₂O₃/SiO₂ (80 Gew.-%/20 Gew-.%) und je 2,5 g Phenothiazin und Hydrochinonmonomethylether vorgelegt. Bei einer Öltemperatur von 200°C und einem Druck von 11 mbar wurden 5 Fraktionen von je 180 g 1-(4-Acetoxyphenyl)ethylacetat (59,7 Gew.-% Roh-APEA aus der Veresterung in 36,7 Gew.-% Essigsäure) jeweils innerhalb von 3 bis 4 h in das heiße Öl eindosiert (0,9 bis 1,2 g/min). Das Destillat jeder Fraktion wurde getrennt gesammelt und analysiert:

| Destillat | Ausbeute an 4-Acetoxystyrol [%] | Umsatz [%] |
|---|---|---|
| 1 | 87,0 | 88,3 |
| 2 | 85,3 | 88,1 |
| 3 | 79,4 | 81,4 |
| 4 | 75,2 | 78,1 |
| 5 | 72,6 | 75,6 |
| Vergleichsbeispiele mit 1-(4-Acetoxyphenyl)ethanol | | |

### Beispiel A

In einen 250 ml-Rührkolben mit Destillationsaufsatz und Magnetrührer wurden 400 g Marlothermöl 20 g Kaliumhydrogensulfat und 4 g 3,5-Di- tert.-butylbrenzcatechin vorgelegt. Bei 220 bis 230°C/20 mbar wurden über 1 h 2,8 g/h 1-(4-Acetoxyphenyl)ethanol zudosiert und bei einer Kopftemperatur von 160°C 122,8 g eines zweiphasiges Destillat abgenommen. Die organische Phase (105,6 g) hatte folgende Zusammensetzung:
- 71,5 %: aus 4-Acetoxystyrol,
- 15,7 %: 1-(4-Acetoxyphenyl)-ethanol.

Im Sumpf verbleibt ein viskoser Rückstand. Die Ausbeute an 4-Acetoxystyrol betrug 49 %.

### Beispiel B

In einen 500 ml-Rührkolben mit einer 30 cm langen Destillationskolonne wurden 200 g Mineralöl, 20 g Dodecylsulfonsäure und 2 g 3,5-Di-tert.-butylbrenzcatechin bei einem Druck von 0,3 mbar auf 230°C aufgeheizt. Innerhalb von 2 h dosierte man 115 g 1-(4-Acetoxyphenyl)ethanol zu und destillierte kontinuierlich bei einer Temperatur von 67 bis 70°C ein zweiphasiges Destillat bestehend aus 69 g organischer Phase und 11 g Wasser ab. Die organische Phase hat folgende Zusammensetzung:
- 47,8 %: 4-Acetoxystyrol,
- 19,3 %: 4-Hydroxystyrol,
- 14,8 %: 1-(4-Acetoxyphenyl)ethanol.

Die Ausbeute an 4-Acetoxystyrol betrug 32 %.

## Patentansprüche

1. Verfahren zur Herstellung von 4-Acetoxystyrol der Formel I 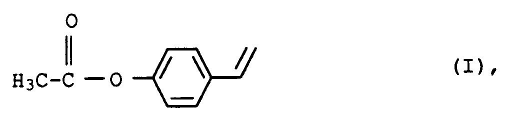 dadurch gekennzeichnet, daß man 1-(4-Acetoxyphenyl)ethylcarboxylate der allgemeinen Formel II 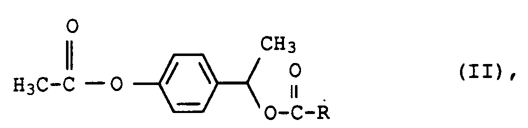 in der R C₁- bis C₁₀-Alkyl, C₃- bis C₆-Cycloalkyl, Aryl oder C₇- bis C₁₀-Aralkyl bedeutet, in Gegenwart eines acidwirkenden Katalysators und eines Polymerisationsinhibitors in einem inerten Wärmeträger bei Temperaturen von 160 bis 250°C und einem Druck von 0,1 bis 300 mbar umsetzt.

2. Verfahren zur Herstellung von 4-Acetoxystyrol I nach Anspruch 1, dadurch gekennzeichnet, daß man Acetoxystyrol während der Umsetzung aus dem Synthesegas destillativ isoliert.

3. Verfahren zur Herstellung von 4-Acetoxystyrol I nach Anspruch 1, dadurch gekennzeichnet, daß man als einen inerten Wärmeträger technische Öle einsetzt.

4. Verfahren zur Herstellung von 4-Acetoxystyrol I nach Anspruch 1, dadurch gekennzeichnet, daß man als einen inerten Wärmeträger Mineralöle, Weißöl oder Silikonöle einsetzt.

5. Verfahren zur Herstellung von 4-Acetoxystyrol I nach Anspruch 1, dadurch gekennzeichnet, daß man als saure Katalysatoren oxidische Feststoffkatalysatoren einsetzt.

6. Verfahren zur Herstellung von 4-Acetoxystyrol I nach Anspruch 1, dadurch gekennzeichnet, daß man als saure Katalysatoren Aluminiumoxid, Siliciumoxid, Titanoxid, Zirkonoxid und/oder Zeolithe einsetzt.

7. Verfahren zur Herstellung von 4-Acetoxystyrol I nach Anspruch 1, dadurch gekennzeichnet, daß man 1-(4-Acetoxyphenyl)ethylacetat oder eine Lösung von 1-(4-Acetoxyphenyl)ethylacetat in Essigsäure einsetzt.

8. Verfahren zur Herstellung von 4-Acetoxystyrol I nach Anspruch 1, dadurch gekennzeichnet, daß man als Polymerisationsinhibitor ein Gemisch aus Hydrochinonmonomethylether und Phenothiazin einsetzt.

## Claims

1. A process for the preparation of 4-acetoxystyrene of the formula I 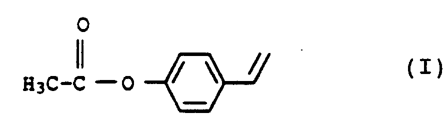 wherein a 1-(4-acetoxyphenyl)ethyl carboxylate of the formula II 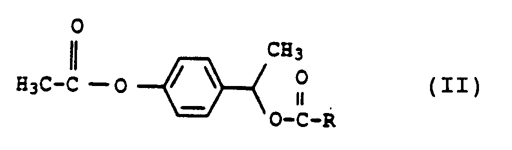 where R is C₁-C₁₀-alkyl, C₃-C₆-cycloalkyl, aryl or C₇-C₁₀-aralkyl, is converted in the presence of an acidic catalyst and of a polymerization inhibitor in an inert heat transfer medium at from 160 to 250°C and from 0.1 to 300 mbar.

2. A process for the preparation of 4-acetoxystyrene I as claimed in claim 1, wherein acetoxystyrene is isolated during the reaction by distillation from the synthesis gas.

3. A process for the preparation of 4-acetoxystyrene I as claimed in claim 1, wherein a technical oil is used as an inert heat transfer medium.

4. A process for the preparation of 4-acetoxystyrene I as claimed in claim 1, wherein a mineral oil, white oil or silicone oil is used as the inert heat transfer medium.

5. A process for the preparation of 4-acetoxystyrene I as claimed in claim 1, wherein the acidic catalyst used is an oxidic solid catalyst.

6. A process for the preparation of 4-acetoxystyrene I as claimed in claim 1, wherein the acidic catalyst used is alumina, silica, titanium oxide, zirconium oxide and/or a zeolite.

7. A process for the preparation of 4-acetoxystyrene I as claimed in claim 1, wherein 1-(4-acetoxyphenyl)ethyl acetate or a solution of 1-(4-acetoxyphenyl)ethyl acetate in acetic acid is used.

8. A process for the preparation of 4-acetoxystyrene I as claimed in claim 1, wherein the polymerization inhibitor used is a mixture of hydroquinone monomethyl ether and phenothiazine.

## Revendications

1. Procédé de préparation du 4-acétoxystyrène de la formule I : 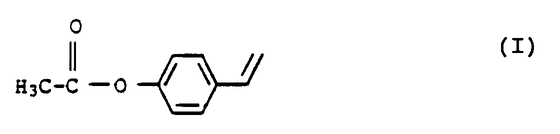 caractérisé en ce que l'on fait réagir des carboxylates de 1-(4-acétoxyphényl)éthyle de la formule générale II : 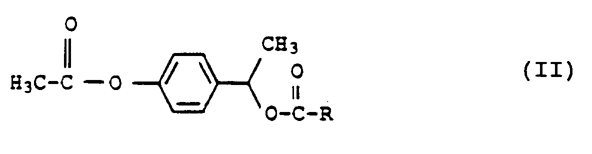 dans laquelle R représente un radical alkyle en C₁-C₁₀, cycloalkyle en C₃-C₆, aryle ou aralkyle en C₇-C₁₀, en présence d'un catalyseur à réaction acide et d'un inhibiteur de polymérisation dans un produit caloporteur inerte, à des températures de 160 à 250°C et sous une pression de 0,1 à 300 mbars.

2. Procédé de préparation du 4-acétoxystyrène I selon la revendication 1, caractérisé en ce que l'on isole l'acétoxystyrène du gaz de synthèse par distillation au cours de la réaction.

3. Procédé de préparation du 4-acétoxystyrène I selon la revendication 1, caractérisé en ce que l'on utilise des huiles industrielles à titre de produit caloporteur inerte.

4. Procédé de préparation du 4-acétoxystyrène I selon la revendication 1, caractérisé en ce que l'on utilise des huiles minérales, de l'huile blanche ou des huiles de silicones à titre de produit caloporteur inerte.

5. Procédé de préparation du 4-acétoxystyrène I selon la revendication 1, caractérisé en ce que l'on utilise des catalyseurs à corps solides oxydiques à titre de catalyseurs acides.

6. Procédé de préparation du 4-acétoxystyrène I selon la revendication 1, caractérisé en ce que l'on utilise l'oxyde d'aluminium, l'oxyde de silicium, l'oxyde de titane, l'oxyde de zirconium et/ou des zéolites à titre de catalyseurs acides.

7. Procédé de préparation du 4-acétoxystyrène I selon la revendication 1, caractérisé en ce que l'on utilise l'acétate de 1-(4-acétoxyphényl)éthyle ou une solution d'acétate de 1-(4-acétoxyphényl)éthyle dans de l'acide acétique.

8. Procédé de préparation du 4-acétoxystyrène I selon la revendication 1, caractérisé en ce que l'on utilise un mélange d'éther monométhylique de l'hydroquinone et de phénothiazine à titre d'inhibiteur de polymérisation.
